# EUROPEAN PATENT APPLICATION

(11) **EP 4 342 990 A1**
(43) Date of publication of application: **27.03.2024**
(21) Application number: 22805029.0
(22) Date of filing: 20.05.2022
(51) Int. Cl.: C12N 15/77, C12P 19/32

(54) **MICROORGANISM PRODUCING PURINE NUCLEOTIDE, AND PURINE NUCLEOTIDE PRODUCTION METHOD USING SAME**

(30) Priority: 21.05.2021 KR 20210065740
(71) Applicant: CJ Cheiljedang Corporation, Seoul 04560 (KR)
(72) Inventor: LEE, Ji Hyun, Seoul 04560 (KR); KWON, Hee Su, Seoul 04560 (KR); KIM, Dae Young, Seoul 04560 (KR); BAE, Hyun-jung, Seoul 04560 (KR); LEE, Ji Hye, Seoul 04560 (KR)
(74) Representative: Regimbeau
(86) International application number: PCT/KR2022/007225
(87) International publication number: WO 2022/245176

(57) **Abstract**

The present disclosure relates to a *Corynebacterium stationis* microorganism producing purine nucleotides in which the activity of a phosphate importer system (Pit system) is enhanced, and a method for producing purine nucleotides using the microorganism.

## Description

### [Technical Field]

The present disclosure relates to a *Corynebacterium stationis* microorganism having purine nucleotide production ability, in which the activity of a phosphate transport system is enhanced, and a method for producing purine nucleotides using the microorganism.

### [Background Art]

Purine nucleotides, i.e., 5'-inosine monophosphate (hereinafter referred to as IMP), 5'-xanthosine monophosphate (hereinafter referred to as XMP), and 5'-guanosine monophosphate (hereinafter referred to as GMP), which are intermediates of the nucleic acid biosynthetic metabolic system, have an important physiological role in the body, and are widely used in foods, pharmaceuticals, *etc.* Specifically, IMP itself is known to impart a beef flavor, and GMP, which is derived from XMP, is known to impart a mushroom flavor. Both materials are known to enhance the taste intensity of monosodium glutamate (MSG), and thus have attracted much attention as a flavor-enriched nucleic acid-based seasoning.

Phosphate, which is a component of purine nucleotides, provides energy necessary for microbial growth, and is essential for the biosynthesis of phospholipids of cell membranes, nucleic acids, and proteins. It also has a major role in the cellular signaling process.

Phosphate is mainly absorbed into cells in the form of inorganic orthophosphate (hereinafter referred to as Pi). Pi influx into microorganisms depends on the function of importers present in the cell membrane. The previously known Pi importers include a Pst system, which is a high-affinity Pi importer system, expression of which is regulated by recognizing the extracellular Pi concentration; a Pit system, which expresses regardless of the Pi concentration and introduces Pi in a mixed form with metal ions; and an antiporter transport system, which introduces glycerol-3-phosphate and glucose-6-phosphate in the form of organophosphate, *etc.*

Several types of methods used in amino acid production by controlling the Pit system among the Pi importer systems have been reported, but the influence of the Pit system on the production of nucleic acids has not been reported. In addition, with respect to increasing amino acid production, it was confirmed that weakening or enhancing the expression of the Pit system leads to different results for each microorganism and amino acid. In the case of amino acid production, it can be confirmed that adjusting the Pi influx system in a specific direction does not have a uniform effect on increasing or decreasing production amount (US 9506094 B2, US 9873898 B2).

### [Disclosure]

### [Technical Problem]

The problem to be solved by the present disclosure is to provide a *Corynebacterium stationis* microorganism having purine nucleotide production ability, in which the activity of a phosphate importer system (Pit system) is enhanced, and a method for producing purine nucleotides using the microorganism.

### [Technical Solution]

An object of the present disclosure is to provide a *Corynebacterium stationis* microorganism having nucleotide production ability, in which the activity of a phosphate importer system (Pit system) is enhanced.

Another object of the present disclosure is to provide a method for producing purine nucleotides, including: culturing the *Corynebacterium stationis* microorganism having purine nucleotide production ability in a medium.

Still another object of the present disclosure is to provide a composition for producing purine nucleotides, including the *Corynebacterium stationis* microorganism having nucleotide production ability; a medium on which the microorganism is cultured; or a combination thereof.

Yet another object of the present disclosure is to provide use of the *Corynebacterium stationis* microorganism having nucleotide production ability for the production of purine nucleotides.

### [Advantageous Effects]

The influx of phosphate, the key component in purine nucleotide production, into microorganisms may be facilitated by enhancing the activity of the phosphate importer according to the present disclosure. As a result, the microorganism having purine nucleotide production ability can efficiently produce purine nucleotides, and can greatly contribute to cost reduction when producing purine nucleotides on an industrial scale.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety and the level of technical field to which the present disclosure belongs and the contents of the present disclosure will be described more clearly.

An aspect of the present disclosure provides a *Corynebacterium stationis* microorganism having purine nucleotide production ability, in which the activity of a phosphate importer system (Pit system) encoded by *pit* gene is enhanced.

As used herein, the term "phosphate importer system" refers to a polypeptide having the function of introducing phosphate into a cell, or a system containing the same. The phosphate importer system may include a phosphate importer. For the purpose of the present disclosure, the phosphate importer system may be a Pit system.

As used herein, the term "phosphate importer" refers to a polypeptide that has a role in intracellular uptake of phosphate. Phosphate is mainly absorbed into cells in the form of inorganic orthophosphate (hereinafter referred to as Pi), and is known to depend on the function of the importers present in the cell membrane. Specifically, the previously known Pi importers include a Pst system, a Pit system, and an antiporter transport system, which introduces glycerol-3-phosphate and glucose-6-phosphate in the form of organophosphate, *etc.,* but are not limited thereto.

As used herein, the term "Pit system" refers to a phosphate transport importer (phosphate transporter, low-affinity; tellurite importer; hereinafter referred to as Pit) that shows low affinity for Pi. The expression of the Pit system does not depend on the concentration of extracellular Pi, and Pi is known to be introduced in the form of a metal cation complex into cells (JOURNAL OF BACTERIOLOGY, Sept. 2001, p. 5008-5014 Vol. 183, No. 17).

The Pit system may include at least one polypeptide encoded by at least one gene selected from *pit, pit1, pit2, pit3, pitA, pitB,* and *pitC,* or Pit, Pit1, Pit2, Pit3, PitA, PitB, and PitC polypeptide, and for the purpose of the present disclosure, the Pit system may be a polypeptide encoded by *pitA* (inorganic phosphate transporter) gene or PitA polypeptide, but is not limited thereto.

The Pit system may include a polypeptide represented by an amino acid sequence of SEQ ID NO: 1, but the system or polypeptide is not limited thereto because the amino acid sequence of an enzyme showing the activity may differ depending on the species or strain of microorganisms.

The PitA polypeptide or polypeptide encoded by the *pitA* gene may consist of a polypeptide represented by the amino acid sequence of SEQ ID NO: 1, but is not limited thereto.

The PitA polypeptide or the polypeptide encoded by the *pitA* gene may have, include, consist of, or consist essentially of the amino acid sequence of SEQ ID NO: 1, but is not limited thereto. More specifically, the SEQ ID NO: 1 may be a polypeptide sequence having the activity of a phosphate importer encoded by the *pitA* gene.

In one embodiment, the polypeptide encoded by the *pitA* gene may include a polypeptide having a homology or identity of 70%, 75%, 80%, 85%, 90%, 95%, 96%, 97%, 98%, or 99% or more to the amino acid sequence of SEQ ID NO: 1, but is not limited thereto.

The amino acid sequence of SEQ ID NO: 1 may be obtained from various data bases, e.g., NCBI GenBank, a known database, *etc.,* but is not limited thereto. In one example, the amino acid sequence may be derived from *Escherichia coli,* but is not limited thereto, and may include any sequence having the same activity as the above amino acid sequence without limitation. In addition, although the polypeptide having the activity of phosphate importer, Pit system or PitA of the present disclosure is defined as the polypeptide including the amino acid sequence of SEQ ID NO: 1, addition of a meaningless sequence upstream or downstream of the amino acid sequence of SEQ ID NO: 1, a naturally occurring mutation, or a silent mutation therein is not excluded, and it will be apparent to those skilled in the art that as long as a polypeptide has activity identical or corresponding to the activity of the polypeptide including the amino acid sequence of SEQ ID NO: 1, it may belong to the polypeptide having the activity of phosphate importer, Pit system or PitA of the present disclosure.

In a specific embodiment, the polypeptide having the activity of phosphate importer, Pit system or PitA of the present disclosure may be a polypeptide consisting of the amino acid sequence of SEQ ID NO: 1, or an amino acid sequence having a homology or identity of 70%, 75%, 80%, 85%, 90%, 95%, 96%, 97%, 98%, or 99% or more thereto.

The polypeptide having the activity of the PitA polypeptide or polypeptide encoded by the *pitA* gene may be a polypeptide consisting of the amino acid sequence of SEQ ID NO: 1, or an amino acid sequence having a homology or identity of 70%, 75%, 80%, 85%, 90%, 95%, 96%, 97%, 98%, or 99% or more thereto.

Additionally, it will be apparent that any polypeptide having an amino acid sequence with deletion, modification, substitution, or addition in part of the sequence may also be included within the scope of the polypeptide of the present disclosure, as long as the amino acid sequence has such homology or identity and exhibits efficacy corresponding to that of the polypeptide.

Although described as "a polypeptide including an amino acid sequence of a specific SEQ ID NO", "a polypeptide consisting an amino acid sequence of a specific SEQ ID NO", or "a polypeptide having an amino acid sequence of a specific SEQ ID NO" in the present disclosure, it is obvious that any polypeptide having an amino acid sequence with deletion, modification, substitution, conservative substitution, or addition in part of the sequence may also be used in the present disclosure, as long as the polypeptide may have an activity identical or corresponding to that of the polypeptide consisting of the amino acid sequence of the corresponding SEQ ID NO: For example, it may include sequence additions that do not alter the function of the protein of the present disclosure at the N-terminus and/or C-terminus of the amino acid sequence, naturally occurring mutations, silent mutations therein, or conservative substitutions.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. Typically, conservative substitutions may have little or no effect on the activity of the polypeptide.

The Pit system may be encoded by one or more genes selected from *pit, pit1, pit2, pit3, pitA, pitB,* and *pitC,* but is not limited thereto. Specifically, the Pit system may be encoded by a polynucleotide including a nucleotide sequence represented by SEQ ID NO: 2, but is not limited thereto due to codon degeneracy. The Pit system may be encoded by a polynucleotide including a nucleotide sequence having a homology or identity of 70%, 75%, 80%, 85%, 90%, 95%, 96%, 97%, 98%, or 99% or more to the nucleotide sequence of SEQ ID NO: 2, but is not limited thereto.

The PitA polypeptide or polypeptide encoded by the *pitA* gene may be encoded by a polynucleotide including the nucleotide sequence represented by SEQ ID NO: 2, but is not limited thereto. The PitA polypeptide or polypeptide encoded by the *pitA* gene may be encoded by a polynucleotide including a nucleotide sequence having a homology or identity of 70%, 75%, 80%, 85%, 90%, 95%, 96%, 97%, 98%, or 99% or more to the nucleotide sequence of SEQ ID NO: 2, but is not limited thereto.

In the present disclosure, the amino acid sequence of SEQ ID NO: 1 may be encoded by a polynucleotide including, for example, the nucleic acid sequence of SEQ ID NO: 2.

As used herein, the "polynucleotide", which is a polymer of nucleotides composed of nucleotide monomers connected in a lengthy chain by a covalently bond, is a DNA or RNA strand having at least a certain length. In the present disclosure, the polynucleotide may encode the polypeptide showing the activity of the phosphate importer or Pit system having the amino acid sequence of SEQ ID NO: 1, but is not limited thereto.

The polynucleotide may include any polynucleotide without limitation as long as it is a polynucleotide encoding the polypeptide having the activity of the phosphate importer, Pit system, or PitA according to the present disclosure. In the present disclosure, the gene encoding the amino acid sequence of the phosphate importer or Pit system may be any one or more genes selected from *pit, pit1, pit2, pit3, pitA, pitB,* and *pitC,* and the gene may be derived from *Escherichia coli,* but is not limited thereto.

Specifically, the polynucleotide encoding the polypeptide having the activity of the phosphate importer, Pit system, or PitA may include a nucleotide sequence encoding the amino acid sequence of SEQ ID NO: 1.

The polynucleotide may undergo various modifications in the coding region without changing the amino acid sequence of the polypeptide, due to codon degeneracy or in consideration of the codons preferred in an organism in which the polypeptide is to be expressed. The polynucleotide may include, for example, the nucleotide sequence of SEQ ID NO: 2, and may consist of a nucleotide sequence having a homology or identity of 80%, specifically 90% or more, more specifically 95% or more, 96% or more, 97% or more, or 98% or more, or even more specifically 99% or more thereto, but is not limited thereto.

Further, a probe which may be prepared from a known gene sequence, for example, any nucleotide sequence which hybridizes with a sequence complementary to all or a part of the nucleotide sequence under stringent conditions to encode the amino acid sequence of SEQ ID NO: 1 may be included without limitation. The "stringent conditions" refer to conditions which allow the specific hybridization between polynucleotides. Such conditions are disclosed in detail in the literature (e.g., J. Sambrook *et al., supra*). For example, the stringent conditions may include conditions under which polynucleotides having high homology or identity, polynucleotides having 40% or more, specifically 90% or more, more specifically 95% or more, 96% or more, 97% or more, 98% or more, and particularly specifically 99% or more hybridize with each other, while polynucleotides having homology or identity lower than the above homology or identity do not hybridize with each other; or may include ordinary washing conditions of Southern hybridization, i.e., washing once, specifically twice or three times, at a salt concentration and a temperature corresponding to 60°C, 1×SSC, 0.1% SDS, specifically 60°C, 0.1×SSC, 0.1% SDS, and more specifically 68°C, 0.1×SSC, 0.1% SSD.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the present disclosure may also include an isolated nucleic acid fragment complementary to the entire sequence as well as a nucleic sequence substantially similar thereto.

Specifically, polynucleotides having a homology or identity may be detected using the hybridization conditions including a hybridization step at a Tₘ value of 55 °C under the above-described conditions. Further, the Tₘ value may be 60 °C, 63 °C, or 65 °C, but is not limited thereto, and may be appropriately adjusted by those skilled in the art depending on the purpose thereof.

The appropriate stringency for hybridizing polynucleotides depends on the length and degree of complementarity of the polynucleotides, and these variables are well known in the art (see Sambrook *et al., supra,* 9.50-9.51, 11.7-11.8).

As used herein, the term "homology" or "identity" refers to the degree of relatedness between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used together with a default gap penalty established by the program being used. Substantially, homologous or identical sequences are generally expected to hybridize to all or at least about 50%, 50%, 60%, 70%, 80%, or 90% of the entire length of the sequences under moderate or highly stringent conditions. Polynucleotides that contain degenerate codons instead of codons in the hybridizing polynucleotides may also be considered.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), J MolBiol. 48:443, as disclosed in Smith and Waterman, Adv. Appl. Math (1981) 2:482. In summary, the GAP program defines the homology, similarity or identity as the value obtained by dividing the number of similarly aligned symbols (*i.e.,* nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity may be determined by comparing sequences via Southern hybridization experiments under defined stringent conditions, and the appropriate hybridization conditions to be defined may be determined by way of a method within the scope of the present disclosure, which is known to those skilled in the art (*e.g.,* J. Sambrook et al., Molecular Cloning, A Laboratory Manual, 2nd Edition, Cold Spring Harbor Laboratory press, Cold Spring Harbor, N.Y., 1989; F. M. Ausubel et al., Current Protocols in Molecular Biology, John Wiley & Sons, Inc., New York).

In one embodiment, the enhancement of the activity of the phosphate importer system may be enhancement of the activity of the polypeptide encoded by *pitA* gene, but is not limited thereto.

In one embodiment, the *pitA* gene of the present disclosure may be a foreign *pitA* gene. In another embodiment, the microorganism of the present disclosure may be one into which a foreign *pitA* gene or a foreign pit polypeptide has been introduced, but is not limited thereto. In addition, it includes those in which the introduced foreign genes or polypeptides have been enhanced. Specifically, the foreign *pitA* gene may be derived from *Escherichia* sp. or *Escherichia coli,* but is not limited thereto.

As used herein, the term "enhancement of polypeptide activity" means that a polypeptide activity is increased, as compared with endogenous activity thereof. The enhancement may be used interchangeably with terms such as activation, up-regulation, overexpression, increase, *etc.* In particular, the terms activation, enhancement, up-regulation, overexpression and increase may include both cases in which an activity not originally possessed is exhibited, or the activity is enhanced compared to the endogenous activity or the activity before modification. The "endogenous activity" refers to activity of a particular polypeptide originally possessed by a parent strain before transformation or a non-modified microorganism when a trait is altered by genetic variation due to a natural or artificial factor. The endogenous activity may also be interchangeably used with "activity before modification". The "enhancement", "up-regulation", "overexpression", or "increase" in the activity of the polypeptide, as compared with the endogenous activity, means that the activity of the polypeptide is enhanced, as compared with activity of a particular polypeptide originally possessed by a parent strain before transformation or a non-modified microorganism.

The enhancement may be achieved by introducing a foreign polypeptide or enhancing the activity and/or concentration (expression level) of an endogenous polypeptide. Whether or not the activity of the polypeptide is enhanced may be confirmed from the activity or expression level of the corresponding polypeptide, or the increase in the amount of a product produced from the corresponding polypeptide.

The enhancement of the activity of the polypeptide can be applied by various methods well known in the art, and is not limited as long as it can enhance the activity of the target polypeptide compared to that of the microorganism before modification. Specifically, genetic engineering and/or protein engineering well known to those skilled in the art, which is a common method of molecular biology, may be used, but the method is not limited thereto (*e.g.,* Sitnicka et al. Functional Analysis of Genes. Advances in Cell Biology. 2010, Vol. 2. 1-16; Sambrook et al., Molecular Cloning 2012, *etc*.).

Specifically, the enhancement of the polypeptide activity of the present disclosure may be achieved by:
1) increasing the intracellular copy number of a polynucleotide encoding the polypeptide;
2) replacing the expression regulatory region of a gene encoding the polypeptide on the chromosome with a sequence having a strong activity;
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g.,* modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

More specifically:
The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.
The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative, or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

Further, the 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration (expression level) of the corresponding polypeptide is increased relative to the activity or concentration of the polypeptide expressed in a wild-type strain or a microorganism before modification, or that the amount of product produced from the polypeptide is increased, but is not limited thereto.

The modification of a part or all of the polynucleotide in the microorganism of the present disclosure may be achieved by (a) homologous recombination using a vector for chromosomal insertion in the microorganism or genome editing using an engineered nuclease (*e.g.,* CRISPR-Cas9), and/or (b) may be induced by light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

In the present disclosure, the polypeptide targeted for the activity enhancement may be a phosphate importer, and specifically, it may be the Pit system or the polypeptide encoded by the *pitA* gene, or the PitA polypeptide. For the purpose of present disclosure, the activity enhancement may be achieved by introducing a foreign Pit system, a foreign polypeptide encoded by the *pitA* gene, or a foreign PitA polypeptide, but is not limited thereto.

For the purposes of the present disclosure, the enhancement of the activity of the phosphate importer system may include introduction of a foreign *pitA* gene or a polypeptide encoded by the foreign *pitA,* but is not limited thereto.

As used herein, the term "introduction of a polypeptide" may mean exhibiting the activity of a specific polypeptide that was not originally possessed by a microorganism or exhibiting improved activity compared to an endogenous activity or activity before modification of the polypeptide. For example, a specific polypeptide may be introduced, a polynucleotide encoding a specific polypeptide may be introduced into a chromosome within a microorganism, or a vector containing a polynucleotide encoding a specific polypeptide may be introduced into the microorganism to exhibit its activity.

As used herein, the term "foreign gene" refers to a non-native (non-natural) gene included in a microorganism. For example, it may be (a) a gene that cannot be found naturally in a microorganism (exogenous), (b) a gene that can be found naturally in a microorganism (endogenous), but the transcription or translation of the gene in the microorganism results in an unnatural amount (greater or lesser amounts compared to the amount naturally present), (c) a polypeptide sequence that is endogenously present in a microorganism, but the sequence of the gene encoding the same is different, and/or (d) a combination of two or more of the above in a microorganism, but is not limited thereto.

For the purpose of the present disclosure, as the pit system does not exist in the *Corynebacterium stationis* microorganism, the foreign gene may be a foreign *pitA* gene, may be a *pitA* gene from *Escherichia* sp., or may be a *pitA* gene derived from *Escherichia coli,* but is not limited thereto.

The vector of the present disclosure may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pBR, pUC, pBluescriptll, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target variant of the present disclosure.

As used herein, the term "strain (or microorganism)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product.

As used herein, the term *"Corynebacterium stationis* microorganism having purine nucleotide production ability" refers to a microorganism that has the ability to produce and accumulate purine nucleotides as a target product within the organism, or to secrete or accumulate purine nucleotides in a medium, when the microorganism is cultured in a medium. In one embodiment, the term "having purine nucleotide production ability" may be used interchangeably with "producing purine nucleotides". The purine nucleotide production ability may be a trait of the wild-type strain of the *Corynebacterium stationis* microorganism, and may be imparted or enhanced through genetic modification.

The microorganism having purine nucleotide production ability of the present disclosure may be a recombinant strain having increased purine nucleotide production ability by enhancing the activity of the phosphate importer system (Pit system) encoded by the *pit* gene. The recombinant strain having increased purine nucleotide production ability may have increased purine nucleotide productivity compared to a natural wild-type microorganism or a microorganism with non-modified phosphate importer system (*i.e.,* a microorganism expressing a wild-type phosphate importer system, a microorganism with no enhanced phosphate importer system, or a microorganism that does not express phosphate importer system), but is not limited thereto. In one example, the microorganism with non-modified phosphate importer system, the target strain to be compared as to whether purine nucleotide production ability is increased may be CJX1664 (KCCM12285P, KR 10-1950141 B1, US 2020-0392478 A1), CJX1665 (KCCM12286P, KR 10-1950141 B1, US 2020-0392478 A1), CJI2332 (KCCM12277P, KR 10-1950141 B1, US 2020-0392478 A1), or CJI2335 (KCCM12278P, KR 10-1956510 B1, EP 3705572 A1), but is not limited thereto.

In one example, the recombinant strain having increased producing ability may have increased purine nucleotide production ability by about 1% or more, specifically about 1% or more, about 2.5% or more, about 5% or more, about 6% or more, about 7% or more, about 8% or more, about 9% or more, about 10% or more, about 11% or more, about 11.5% or more, about 12% or more, or about 13% or more(the upper limit is not particularly limited, for example, about 200% or less, about 150% or less, about 100% or less, about 50% or less, about 40% or less, or about 30% or less), as compared to that of the parent strain before modification or a non-modified microorganism, but is not limited thereto, as long as it has an increased + value compared to the production ability of the parent strain before modification or non-modified microorganism. In another example, the recombinant strain having an increased production ability may have increased purine nucleotide production ability by about 1.01 times or more, about 1.02 times or more, about 1.03 times or more, about 1.05 times or more, about 1.06 times or more, about 1.07 times or more, about 1.08 times or more, about 1.09 times or more, about 1.10 times or more, about 1.11 times or more, about 1.12 times or more, or about 1.13 times or more (the upper limit is not particularly limited, for example, about 10 times or less, about 5 times or less, about 3 times or less, or about 2 times or less) as compared to that of a parent strain before modification or a non-modified microorganism, but is not limited thereto. As used herein, the term "about" refers to a range which includes all of ±0.5, ±0.4, ±0.3, ±0.2, ±0.1, *etc.,* and includes all of the values that are equivalent or similar to those following the values, but the range is not limited thereto.

The microorganism having purine nucleotide production ability of the present disclosure may be a microorganism having a production ability of a target polypeptide or a target product involved in the production of the target polypeptide by including any one or more among the pit system, PitA polypeptide; a polynucleotide encoding the same; and a vector containing the polynucleotide, but is not limited thereto. The microorganism may be a microorganism that naturally has the production ability of the target polypeptide or the target product, or a microorganism, in which the production ability of the target polypeptide or the target product has been imparted to a parent strain that does not have the production ability of the target polypeptide or the target product, but is not limited thereto.

As used herein, the term "non-modified microorganism" does not exclude a strain containing a mutation that may occur naturally in a microorganism, and may refer to a wild-type strain or natural-type strain itself, or a strain before the trait is altered due to genetic modification caused by natural or artificial factors. For example, the non-modified microorganism may refer to a strain in which the phosphate importer system described herein is not introduced or enhanced, or a strain before the enhancement or introduction thereof. The "non-modified microorganism" may be used interchangeably with "strain before modification", "microorganism before modification", "non-mutant strain", "non-modified strain", "non-mutant microorganism", or "reference microorganism".

As used herein, the "purine nucleotide" may be any one or more nucleotides selected from the group consisting of 5'-inosine monophosphate (IMP), 5'-xanthosine monophosphate (XMP), and 5'-guanosine monophosphate (GMP). The IMP is a deaminated adenine compound, and refers to a nucleotide composed of one molecule each of hypoxanthine, ribose, and phosphate. The IMP may be biosynthesized from 5'-phosphoribosyl-1-pyrophosphate (PRPP), and specifically, it may be formed by substituting a pyrophosphate group bound to the first carbon of PRPP with a nitrogen atom and by constituting an imidazole ring and a pyrimidine ring via nine stages, but is not limited thereto. The XMP refers to a nucleotide resulting from dehydrogenation of IMP, and may be synthesized from IMP by inosine-5'-monophosphate dehydrogenase, but is not limited thereto. The GMP refers to a nucleotide having a structure in which a phosphate group forms an ester bond in a ribose of a guanosine molecule. The GMP may be synthesized by adding ammonia molecules to XMP via 5'-guanylic acid biosynthase (GMP synthase), but is not limited thereto.

Another aspect of the present disclosure provides a method for producing purine nucleotides, including culturing a *Corynebacterium stationis* microorganism having purine nucleotide production ability, in which the activity of a phosphate importer system (Pit system) is enhanced, in a medium

The phosphate importer system, Pit system, enhancement, purine nucleotide, and *Corynebacterium stationis* microorganism, *etc.* are as described in other aspects above.

As used herein, the term "cultivation" means that the microorganism is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (i.e., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature of the medium may be in the range from 27°C to 37°C, specifically from 30°C to 33°C, but is not limited thereto. The cultivation may be continued until the production of the useful product reaches a desired level. Specifically, the cultivation may be continued for 20 hours to 120 hours, but is not limited thereto.

Specifically, the culture medium for the microorganism of the genus *Corynebacterium* can be found in the literature ("Manual of Methods for General Bacteriology" by the American Society for Bacteriology (Washington D.C., USA, 1981)).

The purine nucleotides produced by the cultivation of the present disclosure may be released into the medium or remain in the cells.

The method for producing purine nucleotides of the present disclosure may further include a step of preparing the *Corynebacterium stationis* microorganism of the present disclosure, a step of preparing a medium for culturing the microorganism, or a combination thereof (regardless of the order, in any order), for example, prior to the culturing step.

The method for producing purine nucleotides of the present disclosure may further include a step of recovering purine nucleotides from the culture medium (medium on which the culture was grown) or the microorganism (microorganism on which the culture was grown). The recovering step may be further included after the culturing step.

In the recovering step, the desired purine nucleotides may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC or a combination thereof may be used, and the desired purine nucleotides can be recovered from the medium or the microorganisms using suitable methods known in the art.

Further, the method for producing purine nucleotides of the present disclosure may further include a purification step, which may be performed using an appropriate method known in the art. In one example, when the method for producing purine nucleotides of the present disclosure includes both a recovering step and a purification step, the recovering step and the purification step may be performed continuously or intermittently regardless of the order or simultaneously, or may be integrated into one step, but the method is not limited thereto.

Specifically, the method for producing 5'-guanosine monophosphate (GMP) may further include a step of converting 5'-xanthosine monophosphate (XMP) into GMP in the culturing step. In the GMP production method of the present disclosure, the converting step may be additionally included after the culturing step or the recovering step. The converting step can be performed using a suitable method known in the art. For example, the conversion can be performed using coryneform microorganisms, *Escherichia coli,* or XMP aminase (KR 10-0655902 B1), but is not limited thereto.

Still another aspect of the present disclosure provides a composition for producing purine nucleotides, including the *Corynebacterium stationis* microorganism of the present disclosure; a medium on which the microorganism is cultured; or a combination thereof.

The composition of the present disclosure may further include any suitable excipient commonly used in compositions for producing purine nucleotides, and such excipients include, for example, preservatives, wetting agents, dispersing agents, suspending agents, buffers, stabilizers, or isotonic agents, *etc.,* but are not limited thereto.

In the composition of the present disclosure, the microorganism, medium, and purine nucleotide, *etc.* are as described in other aspects above.

Yet another aspect of the present disclosure provides use of the *Corynebacterium stationis* microorganism of the present disclosure for the production of purine nucleotides.

In the use for the production of purine nucleotides, the microorganism and purine nucleotide, *etc.* are as described in other aspects above.

### [Mode for Carrying Out the Invention]

Hereinafter, the present disclosure will be described in detail by way of Examples. However, these Examples are merely preferred Examples provided for illustrative purposes, and it is apparent to those skilled in the art to which the present disclosure pertains that the scope of the present disclosure is not intended to be limited by these Examples.

### Example 1: Preparation of Foreign pitA-Introduced Strain and Evaluation of IMP Productivity

### 1-1: Preparation of Recombinant Vector for Introduction of Foreign pitA Gene and Preparation of IMP-Producing Strain

The pit system does not exist in wild-type strains of *Corynebacterium stationis.* Accordingly, a vector for introducing a foreign Pit system into *Corynebacterium stationis* was prepared. In addition, the CJ1 promoter (Korean Patent No. 10-0620092, SEQ ID NO: 3), a known strong promoter among the promoters of microorganisms of the genus *Corynebacterium,* was conjugated to the foreign *pitA* gene. A vector for replacement with the *fepB* gene, one of the periplasmic binding proteins of microorganisms of the genus *Corynebacterium,* was prepared.

Specifically, in order to prepare an insertion vector capable of deleting the *fepB* gene of *Corynebacterium stationis,* a chromosomal gene of ATCC6872 strain, which is a wild-type *Corynebacterium stationis,* was isolated using a G-spin total DNA extraction mini kit (Cat. No. 17045, Intron) according to a protocol provided in the kit, and PCR was performed using this as a template. As a polymerase, Maxime PCR PreMix (i-pfu) high-fidelity DNA polymerase (Intron) was used. PCR was performed as follows: denaturation at 95°C for 5 minutes, followed by 24 cycles of denaturation at 95°C for 30 seconds, annealing at 54°C for 30 seconds, and polymerization at 72°C for 1 minute and 30 seconds. As a result, two different PCR products (fepBdel-A, fepBdel-B) were obtained. The fepB-A had a size of 1038 bp, and was amplified using SEQ ID NO: 4 and SEQ ID NO: 5 as primers. The fepB-B had a size of 1111 bp, and was amplified using SEQ ID NO: 6 and SEQ ID NO: 7 as primers. Secondary PCR was performed using the two kinds of amplification products as templates by a sewing PCR technique. PCR was performed under the same conditions as above, and as a result, a PCR product of 2131 bp was obtained in which the *fepB* gene is deleted and which includes Spel and Notl restriction enzyme sites in the middle. Thereafter, the amplification products were digested using the restriction sites (fepBdel-A: Hindlll, fepBdel-B: Hindlll) located at both ends, and then cloned into the pDZ vector by T4 ligase activity, and as a result, a pDZ-fepBdel vector for *fepB* gene deletion was obtained. The sequences of the primers used above are as follows.

**[Table 1]**

| SEQ ID NO: | Name | Sequence |
|---|---|---|
| 4 | fepBdel-A-F | CCCAAGCTTCCGGTGTTCAGAATCGCTCCG |
| 5 | fepBdel-A-R | GCGGCCGCAAAGGACTAGTCCTCCGGCAT TCAGTCAGGTC |
| 6 | fepBdel-B-F | CTAGTCCTTTGCGGCCGCCCATTCCGCCCT TCAACCTTCCGCCTAGATTACTTCTC |
| 7 | fepBdel-B-R | CCCAAGCTTGTGCAAGCTGTGGATCGTCTT CC |

A vector, in which the nucleic acid region of the *fepB* gene deleted from the pDZ-fepBdel vector was replaced with a foreign pitA, was prepared as follows. Specifically, the chromosomes of the wild-type *Corynebacterium stationis* ATCC6782 strain and *E coli* MG1655 were isolated, and PCR was performed using the chromosomes as a template and Maxime PCR premix (i-pfu) high-fidelity DNA polymerase (Intron) as a polymerase. PCR was performed as follows: denaturation at 95°C for 5 minutes, followed by 24 cycles of denaturation at 95°C for 30 seconds, annealing at 54°C for 30 seconds, and polymerization at 72°C for 2 minutes and 30 seconds. As a result of PCR, two PCR products (pCJ1-pitA-A, pCJ1-pitA-B) of the *pitA* gene conjugated to the CJ1 promoter were obtained. The pCJ1-pitA-A, which is the PCR product of the promoter region, had a size of 341 bp, and was amplified using SEQ ID NO: 8 and SEQ ID NO: 9 as primers. The pCJ1-pitA-B, which is the PCR product of the *pitA* gene, had a size of 1537 bp, and was amplified using SEQ ID NO: 10 and SEQ ID NO: 11 as primers. Secondary PCR was performed using the two kinds of amplification products as templates by a sewing PCR technique. PCR was performed under the same conditions as above, and as a result, the pitA gene conjugated with the pCJ1 promoter containing the nucleotide sequences homologous to the pDZ-fepBdel vector at both ends was obtained. This gene was cloned into the pDZ-fepBdel vector treated with Spel and Notl restriction enzymes using a homologous recombinase to obtain the pDZ-ΔfepB::pCJ1/pitA(Eco) vector. The sequences of the primers used above are as follows.

**[Table 2]**

| SEQ ID NO: | Name | Sequence |
|---|---|---|
| 8 | pCJ1-pitA-A-F | TTCGACCTGACTGAATGCCGGAGGAACCGC GGGCTTATTCCATTACATG |
| 9 | pCJ1-pitA-A-R | CAAACAAATGTAGCATTTAATCTCCTAGATT GGGTTTCACTCAAGG |
| 10 | pCJ1-pitA-B-F | ACCCAATCTAGGAGATTAAATGCTACATTTG TTTGCTGGCCTG |
| 11 | pCJ1-pitA-B-R | CGGAAGGTTGAAGGCGGATTACAGGAACTG CAAGGAGAGCCAG |

Thereafter, the pDZ-ΔfepB::pCJ1/pitA(Eco) vector obtained above was transformed into each strain of CJI2332, which is an IMP-producing strain (*Corynebacterium stationis* ATCC6872-derived IMP-producing strain, KCCM12277P, Korean Patent No. 10-1950141, US 2020-0392478 A1), and CJI2335, (*Corynebacterium stationis* ATCC6872-derived IMP-producing strain KCCM12278P, Korean Patent No. 10-1956510, EP 3705572 A1) by electroporation to obtain strains containing the CJ1 promoter-pitA gene in the middle of the endogenous *fepB* gene on the chromosome through a second crossover process. The newly inserted CJ1 promoter-pitA gene was confirmed using SEQ ID NO: 12 and SEQ ID NO: 13 as primers, which can amplify the region where both ends of the inserted gene and the *fepB* gene meet. In the same manner as above, two strains, CJI2332_pCJ1/pitA and CJI2335_pCJ1/pitA, which are strains that constitutively overexpress pitA introduced exogenously through the CJ1 promoter, were obtained.

**[Table 3]**

| SEQ ID NO: | Name | Sequence |
|---|---|---|
| 12 | fepBdel-check-F | GACCTGACTGAATGCCGGAGG |
| 13 | fepBdel-check-R | GGAAGGTTGAAGGCGGAGTGG |

### 1-2. Evaluation of IMP Productivity of Foreign pitA-Introduced Strain

To Examine improvement in the IMP productivity of the CJI2332_pCJ1/pitA strain and the CJI2335_pCJ1/pitA strain prepared in Example 1-1, the following experiment was performed.

The CJI2332_pCJ1/pitA, CJI2332, CJI2335_pCJ1/pitA, and CJI2335 was each seeded in 5 mL of a seed medium in an autoclaved test tube having a diameter of 18 mm, and cultured with shaking at a temperature of 30°C for 24 hours, and used as a seed culture broth. 29 mL of a fermentation medium was dispensed in a 250 mL shaking Erlenmeyer flask, and then autoclaved at a temperature of 121°C for 15 minutes. Thereafter, 2 mL of the seed culture broth was seeded therein and cultured for 3 days. The culture conditions were controlled at 170 rpm, 30°C, and pH 7.5. The compositions of the seed medium and the fermentation medium are as follows.

### IMP Seed Medium

1% glucose, 1% peptone, 1% meat extract, 1% yeast extract, 0.25% sodium chloride, 100 mg/L adenine, 100 mg/L guanine, pH 7.2 (based on 1 L of medium)

### IMP Flask Fermentation Medium

0.1% sodium glutamate, 1% ammonium chloride, 1.2% magnesium sulfate, 0.01% calcium chloride, 20 mg/L iron sulfate, 20 mg/L manganese sulfate, 20 mg/L zinc sulfate, 5 mg/L copper sulfate, 23 mg/L L-cysteine, 24 mg/L alanine, 8 mg/L nicotinic acid, 45 µg/L biotin, 5 mg/L thiamine-HCl, 30 mg/L adenine, 1.9% phosphoric acid (85%), 4.2% glucose, 2.4% raw sugar (based on 1 L of medium)

The results of measuring IMP production by way of a method using HPLC after completion of the culture are shown in Table 4 below.

**[Table 4]**

| | Strain | IMP (g/L) | | | IMP Productivity (%) |
|---|---|---|---|---|---|
| | | Flask 1 | Flask 2 | Flask 3 | |
| Control group 1-1 | CJI2332 | 1.0 | 1.1 | 1.0 | 100 |
| Experimental group 1-1 | CJI2332_pCJ1/pitA | 1.2 | 1.2 | 1.1 | 113.0 |
| Control group 1-2 | CJI2335 | 1.1 | 1.1 | 1.0 | 100 |
| Experimental group 1-2 | CJI2335_pCJ1/pitA | 1.2 | 1.2 | 1.2 | 112.5 |

As shown in Table 4, it was confirmed that under the same conditions, the yield of IMP accumulation produced by the CJI2332_pCJ1/pitA strain and the CJI2335_pCJ1/pitA strain increased by about 13% and about 12.5%, respectively, compared to the parent strains CJI2332 and CJI2335. The CJI2332_pCJ1/pitA was named CJI2630 and deposited at the Korean Culture Center of Microorganisms (KCCM), a Depositary Authority, under the Budapest Treaty on April 13, 2021, with Accession No. KCCM12974P.

The CJI2335_pCJ1/pitA was named CJI2631 and deposited at the Korean Culture Center of Microorganisms (KCCM), a Depositary Authority, under the Budapest Treaty on April 13, 2021, with Accession No. KCCM12975P.

### Example 2: Preparation of Foreign pitA-Introduced Strain and Evaluation of XMP Productivity

### 2-1: Preparation of Foreign pitA-Introduced Strain

The pDZ-ΔfepB::pCJ1/pitAA(Eco) was transformed into each strain of CJX1664, which is an XMP-producing strain (*Corynebacterium stationis-derived* XMP-producing strain, KCCM12285P, Korean Patent No. 10-1950141, US 2020-0392478 A1), and CJX1665, which is a purA(G85S) variant strain of CJX1664 (KCCM12286P, Korean Patent No. 10-1950141, US 2020-0392478 A1) by electroporation to obtain CJX1664_pCJ1/pitA strain and CJX1665_pCJ1/pitA strain containing the pCJ1-pitA gene in the middle of the endogenous *fepB* gene on the chromosome through a second crossover process. The newly inserted pCJ1-pitA gene was confirmed using SEQ ID NO: 12 and SEQ ID NO: 13 as primers, which can amplify the region where both ends of the inserted gene and the *fepB* gene meet.

### 2-2. Evaluation of XMP Productivity of Foreign pitA-Introduced Gene

To measure XMP productivity of the CJX1664_pCJ1/pitA strain and the CJX1665_pCJ1/pitA strain prepared in Example 2-1, a flask test was performed. Each of CJX1664, CJX1664_pCJ1/pitA, CJX1665, and CJX1665_pCJ1/pitA was seeded in a 14 mL tube containing 2.5 mL of the following seed medium, and cultured with shaking at 30°C and 170 rpm for 24 hours. 0.7 mL of the seed culture broth was seeded in a 250 mL corner-baffle flask containing 32 mL (24 mL of main medium + 8 mL of separate sterile medium) of the following production medium, and cultured with shaking at 30°C and 170 rpm for 75 hours. The compositions of the seed medium, the main medium, and the separate sterile medium are as follows.

### XMP Flask Seed Medium

30 g/L glucose, 15 g/L peptone, 15 g/L yeast extract, 2.5 g/L sodium chloride, 3 g/L urea, 150 mg/L adenine, 150 mg/L guanine, pH 7.0 (based on 1 L of medium)

### XMP Flask Production Medium (Main Medium)

50 g/L glucose, 10 g/L magnesium sulfate, 100 mg/L calcium chloride, 20 mg/L iron sulfate, 10 mg/L manganese sulfate, 10 mg/L zinc sulfate, 0.8 mg/L copper sulfate, 20 mg/L histidine, 15 mg/L cysteine, 15 mg/L beta-alanine, 100 µg/L biotin, 5 mg/L thiamine, 50 mg/L adenine, 25 mg/L guanine, 15 mg/L niacin, pH 7.0 (based on 1 L of medium)

### XMP Flask Production Medium (Separate Sterile Medium)

18 g/L potassium phosphate monobasic, 42 g/L potassium phosphate dibasic, 7 g/L urea, 5 g/L ammonium sulfate (based on 1 L of medium)

The results of measuring XMP production by way of a method using HPLC after completion of the culture are shown in Table 5 below.

**[Table 5]**

| | Strain | XMP (g/L) | | | XMP Productivity (%) |
|---|---|---|---|---|---|
| | | Flask 1 | Flask 2 | Flask 3 | |
| Control group 2-1 | CJX1664 | 2.5 | 2.5 | 2.4 | 100 |
| Experimental group 2-1 | CJX1664_ pCJ1/pitA | 2.6 | 2.7 | 2.6 | 106.7 |
| Control | CJX1665 | 2.7 | 2.6 | 2.5 | 100 |
| group 2-2 | | | | | |
| Experimental group 2-2 | CJX1665_pCJ1/pitA | 2.8 | 2.8 | 2.7 | 106.4 |

As shown in Table 5 above, the concentration of XMP produced by CJX1664_ pCJ1/pitA and CJX1665_pCJ1/pitA, which are strains that constitutively express pitA of *E coli,* was increased by 6.7% and 6.4%, respectively, compared to the parent strains of CJX1664 and CJX1665.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJI2630 | Accession number given by the
INTERNATIONAL DEPOSITARY AUTHORITY:
KCCM12974P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on April. 13. 2021 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 13. 2021. 한국미생물보존센터장의인

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS [?]
Yurim Bldg., 45, Hongjenae 2ga-gil, Seodaemun-gu, Seoul, 03641, Korea Tel: 82-2-391-0950, 396-0950 Fax: 82-2-392-2859

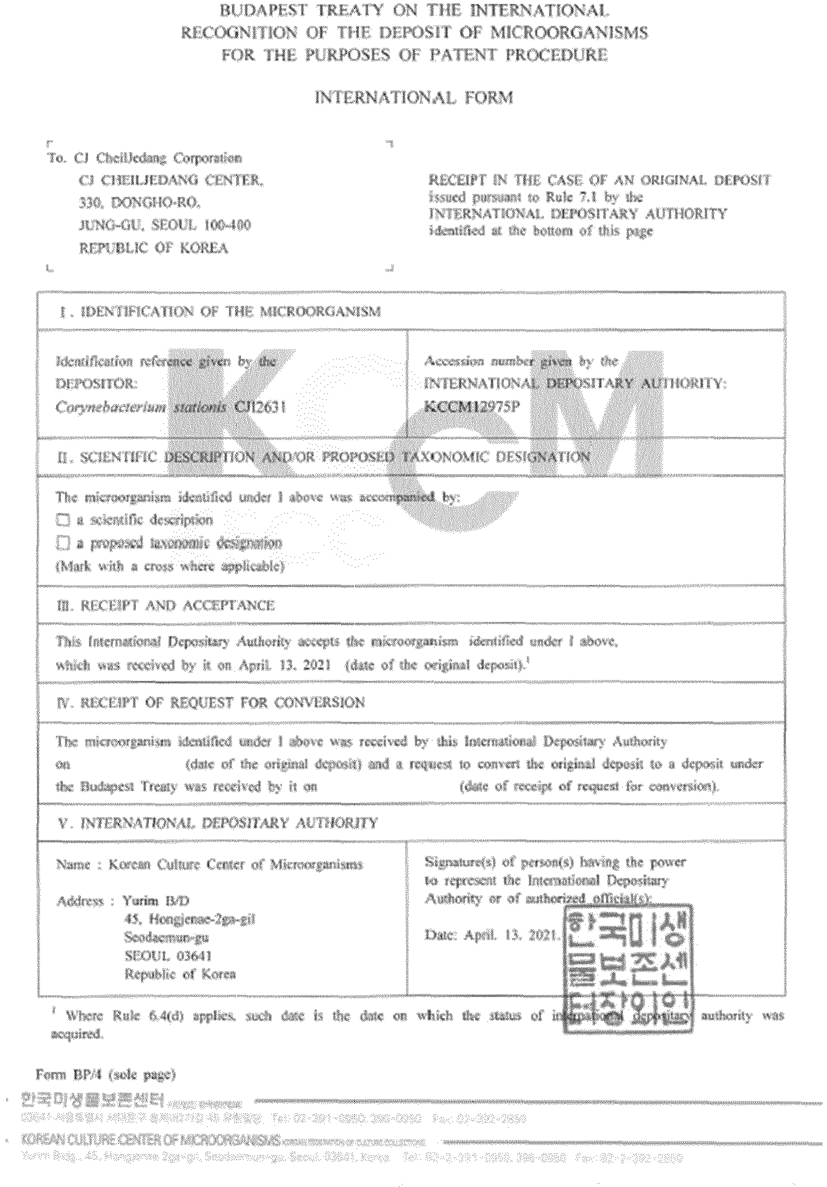
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJI2631 | Accession number given by the
INTERNATIONAL DEPOSITARY AUTHORITY:
KCCM12975P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 13. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 13. 2021.

¹ Where Rule 6.4(d) applies, such date is the date on which the status of international depositary authority was
acquired.

Form BP/4 (sole page)

한국미생물보존센터 [?]
[?]
KOREAN CULTURE CENTER OF MICROORGANISMS [?]
[?]

## Claims

1. A *Corynebacterium stationis* microorganism having purine nucleotide production ability, in which the activity of a phosphate importer system encoded by *pit* gene is enhanced.

2. The microorganism of claim 1, wherein the enhancement of the activity of the phosphate importer system is enhancement of the activity of a polypeptide encoded by *pitA* gene

3. The microorganism of claim 2, wherein the *pitA* gene is a foreign *pitA* gene.

4. The microorganism of claim 3, wherein the foreign *pitA* gene is derived from *E coli.*

5. The microorganism of claim 2, wherein the polypeptide encoded by *pitA* gene comprises a polypeptide having an identity of 99% or more to an amino acid sequence of SEQ ID NO: 1.

6. The microorganism of claim 1, wherein the purine nucleotide is any one or more selected from the group consisting of 5'-inosine monophosphate, 5'-xanthosine monophosphate, and 5'-guanosine monophosphate.

7. A method for producing purine nucleotides, comprising culturing the microorganism of any one of claims 1 to 6 in a medium.

8. The method of claim 7, wherein the method further comprises recovering purine nucleotides from the cultured microorganism or the medium.

9. The method of claim 7, wherein the purine nucleotide is any one or more selected from the group consisting of 5'-inosine monophosphate, 5'-xanthosine monophosphate, and 5'-guanosine monophosphate.

10. A composition for producing purine nucleotides, comprising a *Corynebacterium stationis* microorganism having purine nucleotide production ability, in which the activity of a phosphate importer system encoded by *pit* gene is enhanced; a medium on which the microorganism is cultured; or a combination thereof.

11. Use of a *Corynebacterium stationis* microorganism having purine nucleotide production ability, in which the activity of a phosphate importer system encoded by *pit* gene is enhanced, for the production of purine nucleotides.
